# EUROPÄISCHE PATENTSCHRIFT

(11) **EP 1 154 742 B1**
(45) Veröffentlichungstag und Bekanntmachung des Hinweises auf die Patenterteilung: **28.06.2006**
(21) Anmeldenummer: 00991629.7
(22) Anmeldetag: 22.12.2000
(51) Int. Cl.: A61F 9/01

(54) **VORRICHTUNG FÜR DIE PHOTOREFRAKTIVE KERATEKTOMIE DES AUGES MIT ZENTRIERUNG**
DEVICE USED FOR THE PHOTOREFRACTIVE KERATECTOMY OF THE EYE USING A CENTERING METHOD
DISPOSITIF POUR LA KERATECTOMIE PHOTOREFRACTIVE DE L'OEIL AVEC CENTRAGE

(30) Priorität: 22.12.1999 DE 19962107
(43) Veröffentlichungstag der Anmeldung: 21.11.2001
(73) Patentinhaber: WaveLight AG, 91058 Erlangen (DE)
(72) Erfinder: DONITZKY, Christof, 90542 Eckental (DE); LÖFFLER, Joachim, 90562 Heroldsberg (DE)
(74) Vertreter: von Hellfeld, Axel
(86) Internationale Anmeldenummer: PCT/EP2000/013173
(87) Internationale Veröffentlichungsnummer: WO 2001/045606

(56) Entgegenhaltungen:
- EP-A- 0 770 370
- US-A- 4 848 340
- US-A- 5 604 818
- US-A- 5 644 642

## Beschreibung

Die Erfindung betrifft eine Vorrichtung für die photorefraktive Keratektomie des Auges mit einem UV-Laserstrahl (UV) zum Ablatieren von Teilen der Kornea, und einem Fixierlichtstrahl, der eine Wellenlänge im sichtbaren Bereich hat und auf das Auge gerichtet ist.

Bei der photorefraktiven Keratektomie (PRK) (englisch: photorefractive keratectomy) wird eine Fehlsichtigkeit des menschlichen Auges dadurch korrigiert, daß die Kornea teilweise neu geformt wird. Ein besonderes, zur Zeit erheblich an Bedeutung gewinnendes Verfahren der PRK ist LASIK. Bei dem LASIK-Verfahren wird ein Deckel ("Flap") in die Kornea geschnitten und aufgeklappt. Sodann wird mit dem UV-Laserstrahl (üblicherweise ein Excimer-Laserstrahl mit einer Wellenlänge von 193 nm) auf die freiliegenden (durch den Deckel freigegebenen) Teile der Kornea gerichtet, um dort Material abzutragen (zu ablatieren). Nach der gewünschten Ablation wird der Dekkel wieder zugeklappt und verheilt mit der Kornea.

Die vorliegende Erfindung betrifft allgemein PRK und insbesondere das LASIK-Verfahren.

Bei der PRK bzw. LASIK hängt das Ablationsprofil, also die örtliche Verteilung der Stärke der zu ablatierenden Korneabereiche, von der Art des zu korrigierenden Sehfehlers ab. Bei der Korrektur einer Myopie oder einer Hyperobie ist das Ablationsprofil rotationssymmetrisch; bei der Korrektur des Astigmatismus ist das Ablationsprofil asymmetrisch. Auch bei Korrektur von Sehfehlern höherer Ordnung (Wellenfrontkorrektur, Aberrationskorrektur) gilt letzteres.

Für alle vorgenannten Korrekturen ist es aber in aller Regel erforderlich, eine Achse zu definieren, in bezug auf die die Ablation zentriert wird. Die Ablation entsprechend dem Ablationsprofil erfolgt gemäß dem Fachmann bekannten Algorithmen, d. h. die UV-Strahlung wird entsprechend dem Algorithmus zeitlich und örtlich verteilt auf die zu verändernde Korneafläche gerichtet. Dabei benötigt der Algorithmus eine Achse, die das Zentrum des Ablationsprofils definiert, d. h. die Achse geht durch die zu bearbeitende Korneafläche und das Ablationsprofil wird in bezug auf die Schnittstelle zwischen diese Achse und der Korneafläche dargestellt. Damit wird eine Zentrierung des PRK- bzw. LASIK-Verfahrens ermöglicht, worum es bei der vorliegenden Erfindung geht.

Die Zentrierung chirurgischer Eingriffe an der Kornea wird dadurch erschwert, daß das menschliche Auge kein zentriertes optisches System ist, d. h. es gibt keine gemeinsame optische Achse von Kornea, Vorderkammer, Linse, Glaskörperraum und Fovea des Auges. H. UOZATO und G. L. GUYTON beschreiben allgemein das Problem der Zentrierung bei chirurgischen Eingriffen an der Kornea, vgl. American Journal of Ophtalmology 103:261 - 275, März 1987. Dieser Stand der Technik wird nachfolgend als bekannt vorausgesetzt. Dort wird vorgeschlagen und erläutert, den Mittelpunkt der Eingangspupille des Auges und nicht die sog. "visuelle Achse" für die Zentrierung chirurgischer Eingriffe an der Kornea zu wählen. Es wird eine sog. optische Gesichtslinie ("line of sight") definiert, die den Fixationspunkt mit dem Mittelpunkt der Eingangspupille verbindet. Die vorstehend definierte Gesichtslinie ist, wie gesagt, optisch und nicht geometrisch zu verstehen. Der Fixationspunkt entspricht dem oben genannten Fixierlichtstrahl, ist also derjenige Punkt, den der Patient anschaut. H. UOZATO et al. (siehe oben) schlagen den Schnittpunkt der genannten Gesichtslinie mit der Vorderfläche der Kornea als Zentrum für den chirurgischen Eingriff an der Kornea vor. Von diesem Stand der Technik geht die vorliegende Erfindung aus, d. h. es wird der Durchstoßungspunkt der "line of sight" (Gesichtslinie) durch die korneale Vorderfläche als das Zentrum der refraktiven Korrektur gewählt. Bei dem LASIK-Verfahren ist der Begriff "korneale Vorderfläche" in dem Sinne zu verstehen, daß die freiliegende Fläche der Kornea (die z. B. im Stroma liegen kann) den genannten Durchstoßungspunkt bildet.

Theoretisch könnte zur Ermittlung des genannten Durchstoßungspunktes wie folgt vorgegangen werden: Die Gesichtslinie ("line of sight") verbindet den Fixationspunkt und die Fovea. Das Fixierlicht bildet auf der Korneavorderfläche einen Reflex (d. h. es wird reflektiert), den sog. Streulicht-/Fresnelreflex. Dieser Streulicht-/Fresnelreflex geht also vom Durchstoßungspunkt aus und ermöglicht somit dessen Lagebestimmung. Der Streulicht-/Fresnelreflex ist nicht mit dem Purkinje-Sanson Bild zu verwechseln (vgl. den eingangs genannten Stand der Technik). Mit diesem Verfahren könnte also der Streulicht-/Fresnelreflex des Fixierlichtstrahls als Markierung des Ablationszentrums gewählt werden. Allerdings ist das Bild des Streulicht-/Fresnelreflexes extrem lichtschwach und wird außerdem vom Purkinje-Sanson Bild überstrahlt, so daß die Messung nach diesem Verfahren äußerst schwierig wäre.

Die WO 95/27453 beschreibt die Verwendung eines Infrarotlaserstrahls, der koaxial zum eigentlichen Ablationsstrahl (dem Excimerlaserstrahl) auf die Kornea gerichtet wird. Vor jedem Ablationspuls wird die Position des Korneavorderflächenreflexes dieses IR-Strahls mit einer sog. Eye-Tracking-Kamera ermittelt. Über eine Bildauswertung erfolgt ein Vergleich zwischen einer Soll- und einer Istposition dieses Reflexes relativ zu einem Bezugspunkt des Eye-Tracking. Weicht die ermittelte Istposition von der Sollposition ab, wird versucht, über ein Stellglied (den Scanner) diesen Unterschied auszugleichen. Gelingt dies nicht, erfolgt keine Freigabe des Ablationspulses. Bei diesem Stand der Technik erfolgt keine Lokalisierung des Punktes, in dem der Fixierlichtstrahl durch die Vorderfläche der Kornea tritt. Deshalb kann bei diesem Stand der Technik auch nicht dieser Punkt als Zentrum für die nachfolgende refraktive Korrektur der Kornea gewählt werden.

Die WO 95/28879 und WO 95/28989 beschreiben Verfahren zur Bestimmung der Augenposition mittels vier IR-Dioden, welche an einer optischen Grenzfläche am oder im Auge unterschiedlich stark reflektiert werden. Aufgrund der gemessenen reflektierten Intensitäten der Strahlung erfolgt eine Berechnung der Augenposition.

EP 0 770 370 A2 offenbart eine Vorrichtung zum Entfernen von Korneagewebe mittels eines Laserstrahls im Infrarotbereich. Um zu erreichen, dass ein Patient das zu behandelnde Auge nicht bewegt, wird ein Fixierlichtstrahl verwendet. Die Fixation des Fixierlichtstrahls durch den Patienten soll gewährleisten, dass das Auge korrekt positioniert wird bzw. bleibt. Um die aktuelle Position des Auges zu ermitteln, wird eine Eye-Tracker verwendet, der die Augenposition anhand der Position des Limbus' ermittelt. Um von dem Eye-Tracker zu beobachtende Bereiche besser auszuleuchten, wird ein Beleuchtungsstrahl verwendet. Der Beleuchtungsstrahl ist so auf das Auge gerichtet, dass Reflexionen des Beleuchtungsstrahls an dem Auge nicht von dem Eye-Tracker erfasst werden (können). Es ist weder vorgesehen, die Stelle zu ermitteln, an der der Fixierlichtstrahl in das Auge eintritt, noch die Lage einer Reflexion des Beleuchtungsstrahls auf dem Auge zu bestimmen.

Der Erfindung liegt die Aufgabe zugrunde, eine Vorrichtung und ein Verfahren bereitzustellen, mit dem in zuverlässiger Weise eine Zentrierung bei der photorefraktiven Keratektomie, insbesondere LASIK, möglich ist.

Erfindungsgemäß wird dies mit einer Vorrichtung gemäß Anspruch 1 erreicht, bei der zusätzlich zum UV-Laserstrahl für die Ablation an der Kornea und dem Fixierlichtstrahl, der für den Patienten im sichtbaren Bereich liegen muß, ein Zentrierlichtstrahl vorgesehen ist, der koaxial mit dem Fixierlichtstrahl verläuft, der eine andere Wellenlänge als der Fixierlichtstrahl hat, und einen punktförmigen Reflex auf der Korneavorderfläche erzeugt wobei Mittel vorgesehen sind zum Messendes Streulicht-/Fresnelreflexes des Zentrierlichtstrahls auf der Vorderfläche der Kornea.

Beim LASIK-Verfahren ist unter der "Vorderfläche der Kornea" die freiliegende Kornea-Fläche zu verstehen, nach Aufklappen des Deckels (Flap).

Dadurch, daß für den Zentrierlichtstrahl eine andere Wellenlänge als für den Fixierlichtstrahl gewählt wird, ist es möglich, beide Strahlen beim Vermessen des Reflexes voneinander zu trennen und zu diskriminieren, so daß das Fixierlicht die Messung nicht mehr stört.

Bevorzugt hat der Zentrierlichtstrahl eine Wellenlänge im Infrarotbereich, insbesondere im Bereich von 800 bis 1100 nm.

Eine besonders bevorzugte Ausgestaltung der Erfindung sieht vor, daß eine Kamera zum Messen der Lage des Streulicht-/Fresnelreflexes des Zentrierlichtes eingesetzt wird. Eine derartige Kamera ist bei Vorrichtungen für die photorefraktive Keratektomie des Auges häufig bereits aus anderen Gründen vorgesehen, insbesondere zum sog. Eye-Tracking. Die Technik des Eye-Tracking ist z. B. in der DE 197 02 335 C1 beschrieben, dieser Stand der Technik wird nachfolgend ebenfalls als bekannt vorausgesetzt. Beim "Eye-Tracking" wird danach wie folgt vorgegangen: Bei der PRK, insbesondere bei LASIK, kommt es darauf an, daß die relative Position von ablatierendem Laserstrahl und Auge genau bekannt ist. Bei der optischen Fixierung, die auch bei der vorliegenden Erfindung bevorzugt eingesetzt wird, wird der Patient angehalten, genau auf den durch einen Fixierlichtstrahl definierten Punkt zu schauen, damit das Auge während der gesamten chirurgischen Operation immer die gleiche Position einnimmt. Allerdings gelingt dies dem Patienten in der Regel nicht, jedenfalls nicht mit hinreichender Zuverlässigkeit, so daß es zu Bewegungen des Auges kommt, die den gesamten Ablationsvorgang massiv beeinträchtigen könnten. Beim "Eye-Tracking" werden die Bewegungen des Auges ermittelt, um dann den für die Ablation verwendeten Laserstrahl entsprechend den Augenbewegungen zu steuern (nachzuführen). Der vorstehend genannten Stand der Technik beschreibt, wie beim "Eye-Tracking" in schneller Folge Bilder des Auges mittels einer Kamera (Festkörperkamera, CCD) aufgenommen und verarbeitet werden. Aus aufeinanderfolgenden Bildern kann eine Veränderung der Position des Auges ermittelt werden und entsprechend der Augenbewegung wird dann der Ablationslaserstrahl mit geeigneten Strahlführungseinrichtungen, (z. B. einem galvanometrischen Scanner) nachgeführt. Der genannte Stand der Technik lehrt auch, zur Ermittlung einer Bewegung der Kornea das Zentrum der Pupille zu ermitteln und dessen Bewegung festzustellen. Bei dieser fotografischen Auswertung wird in der Regel das Auge mit IR-Strahlung beleuchtet und das Bild des Auges, insbesondere der Iris mit der Pupille, mit der Kamera aufgenommen, um es auszuwerten. Diese IR-Strahlung, mit der das Auge für den vorstehend genannten Zweck beleuchtet wird, kann zusammen mit der vorliegenden Erfindung eingesetzt werden und wird nachfolgend nicht besonders erwähnt und beschrieben. Wenn auch der Zentrierlichtstrahl Wellenlängen im IR-Bereich hat, können mittels geeigneter teildurchlässiger Spiegel die vom Auge reflektierten IR-Strahlen so umgelenkt und von Reflexionen anderer Wellenlängen getrennt werden, daß mittels der Kamera und einem daran angeschlossenen Rechner sowohl die Lage der Pupille, insbesondere deren geometrischer Mittelpunkt, als auch die Lage des Streulicht-/Fresnelreflexes ermittelt werden können. Es ist also möglich, eine Korellation zwischen dem zu suchenden Ablationszentrum und dem geometrischen Pupillenzentrum herzustellen, also etwa den Vektor vom geometrischen Pupillenzentrum zum genannten Reflex zu bilden und ausgehend von diesem Vektor dann nachfolgend bei der Ablation das Ablationszentrum für die Durchführung des Ablationsalgorithmus zu definieren. Die Meßgenauigkeit bezüglich des Vektors kann dann z. B. dadurch verbessert werden, daß über mehrere Einzelmessungen ein Mittelwert gebildet wird. Es ist auch möglich, den Vektor für unterschiedliche Pupillendurchmesser zu ermitteln und darüber dann eine Mittelung durchzuführen.

Nachfolgend wird ein Ausführungsbeispiel der Erfindung anhand der Zeichnung näher erläutert. Es zeigt:
- Figur 1: schematisch eine Vorrichtung für die photorefraktive Keratektomie des Auges;
- Figuren 2 und 3: Einzelheiten bestimmter Bilder bzw. Reflexe eines Auges, wie sie bei einer Vorrichtung gemäß Figur 1 auftreten.

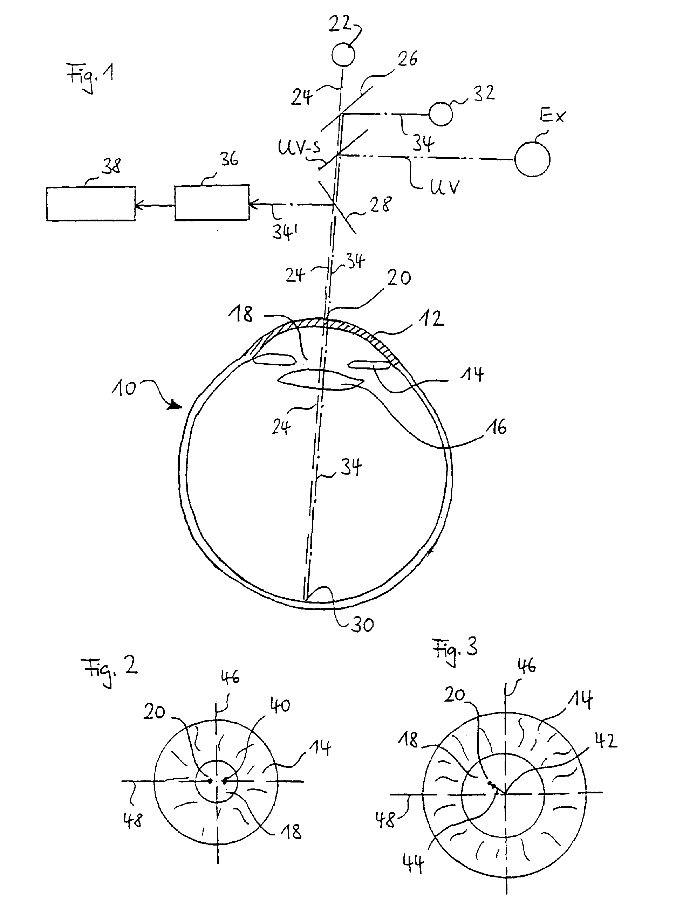

Das in Figur 1 schematisch gezeigte Auge 10 hat eine Kornea 12, eine Iris 14, eine Linse 16 und eine Pupille 18.

Eine als solches bekannte Fixierlichtquelle 22 gibt einen Fixierlichtstrahl 24 ab, der an der Stelle 20 die Vorderfläche der Kornea 12 durchstößt. Die Wellenlänge des Fixierlichtstrahls 24 ist so, daß sie für den Patienten sichtbar ist, liegt also z. B. im grünen Bereich des Spektrums. Als Fixierlichtquelle 22 wird üblicherweise eine Diode verwendet. Der Fixierlichtstrahl 24 ist ortsfest und der Patient ist angehalten, die ihm punktförmig erscheinende Fixierlichtquelle zu fixieren.

Ein Excimerlaser Ex emittiert den eigentlichen Ablationsstrahl, also den Strahl, mit dem die Kornea 12 neu geformt wird. Dieser Ablationsstrahl UV (z.B. 193 nm) wird über einen Spiegel UV-S umgelenkt und gemäß einem Ablationsalgorithmus über die Kornea 12 geführt, so daß das gewünschte Ablationsprofil abgetragen wird. Der Ablationsstrahl ist also nicht ortsfest. Die Mittel zum Bewegen ("scannen") des Ablationsstrahls sind als solches bekannt und in der Figur nicht näher dargestellt.

Der Fixierlichtstrahl 24 tritt durch die Kornea und die Pupille 18 und wird auf die Fovea abgebildet. Er wird deshalb auch als "Gesichtslinie" ("line of sight") bezeichnet. Diese Gesichtslinie verbindet also objektseitig den Fixationspunkt (also den Punkt der Fixierlichtquelle 22) mit dem Zentrum der Eintrittspupille. Die "Eintrittspupille" ist das virtuelle Bild der realen Pupille, das ein Betrachter beim Blick auf das Auge sieht.

Die Stelle 20, an der der Fixierlichtstrahl 24 die Vorderfläche der Kornea 12 durchstößt, wird als Zentrum für die Ablation gewählt, d. h. das Ablationsprofil, gemäß dem der Ablationsstrahl UV über die Kornea 12 geführt ("gescannt") wird, wird auf den Punkt 20 zentriert, in dem der Fixierlichtstrahl 24 die freiliegende Vorderfläche der Kornea 12 durchstößt. Beim LASIK-Verfahren ist die Vorderfläche der Kornea in diesem Sinne die freiliegende Fläche, nach Aufklappen des sog. Deckels (Flap). Um den Durchstoßungspunkt 20 auf der Kornea 12 zu ermitteln, wird eine Zentrierlichtquelle 32 verwendet, die beim dargestellten Ausführungsbeispiel einen Laserstrahl im Infrarotbereich emittiert. Dieser Zentrierlichtstrahl 34 wird über einen teildurchlässigen Spiegel 26 koaxial mit dem Fixierlichtstrahl 24 auf die Kornea 12 gerichtet. In der Figur sind der Fixierlichtstrahl 24 und der Zentrierlichtstrahl 34 parallel nebeneinander gezeichnet, jedoch verlaufen sie tatsächlich koaxial, d. h. auf einer gemeinsamen Zentralachse. Dies bedeutet, daß der während der Operation ortsfeste Zentrierlichtstrahl 34 auch am Durchstoßungspunkt 20 durch die Vorderfläche der Kornea 12 tritt. Beim Ausführungsbeispiel hat der Zentrierlichtstrahl 34 eine Wellenlänge im infraroten Bereich, z. B. im Bereich von 800 bis 1100 nm. Wichtig ist, daß der Zentrierlichtstrahl 34 eine Wellenlänge hat, die verschieden ist von der Wellenlänge des Fixierlichtstrahls 24, so daß Reflexe und Bilder, die von beiden Strahlen erzeugt werden, voneinander diskriminiert werden können, d. h. aufgrund der unterschiedlichen Wellenlängen ist es möglich, einen Reflex des Zentrierlichtstrahls 34 auf der Vorderfläche der Kornea 12 ohne Störung durch den Fixierlichtstrahl zu vermessen. Dementsprechend wird der Durchstoßungspunkt 20 dadurch gemessen, daß der Streulicht-/Fresnelreflex des Zentrierlichtstrahls auf der Korneavorderfläche gemessen wird. Hierzu dient ein teildurchlässiger Spiegel 28, der den Streulicht-/Fresnelreflex 34' des Zentrierlichtstrahls auf eine Kamera 36 lenkt. Die Kamera 36 ist beim dargestellten Ausführungsbeispiel auch aus anderen Gründen Teil der Vorrichtung, nämlich als sog. "Eye-Tracking-Kamera" (vgl. DE 197 02 335 und den darin genannten Stand der Technik).

Die Verwendung eines besonderen Zentrierlichtstrahls 34 für die Bestimmung des Durchstoßungspunktes 20 der ortsfesten Strahlung auf der Korneavorderfläche hat im Vergleich zur Verwendung des Fixierlichtstrahls 24 hierfür den Vorteil, daß eine relativ lichtstarker und nicht von anderen Bildern überstrahlter Reflex mittels der Kamera 36 und eines nachgeschalteten Auswerterechners 38 ausgewertet werden kann. Auch wird der Streulicht-/Fresnelreflex des Fixierlichtes selbst vom Purkinje-Sanson Bild überstrahlt, so daß dieser Reflex schwer auszuwerten ist.

Die Kamera 36 und der Rechner 38, in den die Kamera-Meßwerte eingegeben werden, bilden ein sog. Eye-Tracking-System (vgl. den oben genannten Stand der Technik). Hierzu wird das Auge mit unabhängiger Strahlung, z.B. IR-Strahlung (nicht gezeigt) beleuchtet und z. B. die Pupille 18 mittels ihres Randes vermessen, um insbesondere das geometrische Zentrum der Pupille (der sog. "Pupillenschwerpunkt") zu ermitteln. Daneben vermißt nun das System aus Kamera 36 und Rechner 38 auch die Position des Streulicht-/Fresnelreflexes des Zentrierlichtstrahls 34 auf der Vorderfläche der Kornea 12, d. h. an der Stelle des Durchstoßungspunktes 20. Die Kamera 36 ist somit beim dargestellten Ausführungsbeispiel IR-empfindlich. Bevorzugt ermittelt das System aus Kamera 36 und Rechner 38 die relative Position zwischen Durchstoßungspunkt 20 und geometrischem Zentrum ("Schwerpunkt") der Pupille 18. Dies ist in den Figuren 2 und 3 erläutert:

Figur 2 zeigt die Iris 14 und die Pupille 18 des Auges in schematischer Draufsicht. Das virtuelle Bild 40 der Lichtquelle (hier 22 und/oder 32, je nach gemessener Wellenlänge) wird in der Literatur als das "erste Purkinje-Sanson Bild" bezeichnet. Das geometrische Zentrum der Pupille 18 ist der Schnittpunkt der beiden Achsen 46 und 48. Figur 2 zeigt auch den Streulicht-/Fresnelreflex des Zentrierlichtstrahls 34 auf der Vorderfläche der Kornea 12, d. h. den Durchstoßungspunkt 20. Figur 3 zeigt in etwas vergrößerter Darstellung ebenfalls diesen wie oben beschrieben gemessenen Durchstoßungspunkt 20, sowie den geometrischen Mittelpunkt 42 der Pupille 18 sowie den Vektor 44, der den geometrischen Pupillenmittelpunkt 42 und den Durchstoßungspunkt 20 verbindet. Bei (ungewollten) Bewegungen des Auges ermittelt das Eye-Tracking-System aus Kamera 36 und Rechner 38 in an sich bekannter Weise die jeweilige Augenstellung, d. h. deren Abweichung von der gewünschten Normalstellung gemäß Figur 1 und entsprechend wird auch der Ablationsstrahl UV nachgeführt, d. h. an die tatsächliche Augenstellung angepaßt, entsprechend dem Ablationsprofil. Der Vektor 44 gibt für die Ablation das Zentrum vor und setzt dieses Zentrum in bezug zu dem geometrischen Pupillenmittelpunkt (= "Schwerpunkt der Pupille), den das Eye-Tracking-System sowieso in an sich bekannter Weise ermittelt.

Zur Verbesserung der Meßgenauigkeit ist es möglich, den Vektor 44 durch Mittelwertbildung über eine Vielzahl von Einzelmessungen zu berechnen. Auch kann der Vektor-bei unterschiedlichen Pupillendurchmessern bestimmt werden, um hierüber wiederum eine Mittelwertbildung durchzuführen.

## Patentansprüche

1. Vorrichtung für die photorefraktive Keratektomie des Auges mit
- einem UV-Laserstrahl (UV) zum Ablatieren von Teilen der Kornea,
- einem Fixierlichtstrahl (24), der eine Wellenlänge im sichtbaren Bereich hat und auf das Auge gerichtet ist,
- eine Einrichtung zum Ermitteln des Punktes (20), in dem der Fixierlichtstrahl (24) durch die Vorderfläche der Kornea (12) tritt, mit
- einem Zentrierlichtstrahl (34), der koaxial mit dem Fixierlichtstrahl (24) auf das Auge gerichtet ist, eine andere Wellenlänge hat als der Fixierlichtstrahl und der einen punktförmigen Streulicht-/Fresnelreflex auf der Vorderfläche der Kornea (12) erzeugt, und
- Mitteln (28, 36) zum Messen der Lage des punktförmigen Streulicht-/Fresnelreflexes des Zentrierlichtstrahls (34) auf der Vorderfläche der Kornea (12).

2. Vorrichtung nach Anspruch 1, wobei
der Zentrierlichtstrahl (34) eine Wellenlänge im IR-Bereich hat.

3. Vorrichtung nach Anspruch 2, wobei
der Zentrierlichtstrahl (34) eine Wellenlänge im Bereich von 800 bis 1100 nm hat.

4. Vorrichtung nach einem der vorhergehenden Ansprüche, wobei
die Mittel zum Messen der Lage (20) des Streulicht-/Fresnelreflexes des Zentrierlichtstrahls (34) eine Kamera (36) aufweisen.

5. Vorrichtung nach Anspruch 4, wobei
die Kamera (36) an einen Rechner (38) angeschlossen ist, der aus den Meßdaten das geometrische Pupillenzentrum (42) und die Lage (20) des Streulicht-/Fresnelreflexes des Zentrierlichtstrahls (34) auf der Korneavorderfläche ermittelt.

## Claims

1. A device used for the photorefractive keratectomy of the eye, comprising
- a UV laser beam (UV) for ablating parts of the cornea;
- a fixation light beam (24) which has a wavelength in the visible region and is directed onto the eye;
- means for determining the point (20) at which the fixation light beam (24) passes through the front face of the cornea (12), comprising
- a centring light beam (34) which is directed onto the eye coaxially with the fixation light beam (24), has a wavelength different from that of the fixation light beam, and generates a point scattered light/Fresnel reflex on the front face of the cornea, and
- means (28, 36) for measuring the position (20) of the point scattered light/Fresnel reflex of the said centring light beam (34) on the front face of the cornea (12).

2. The device according to claim 1, wherein the centring light beam (34) has a wavelength in the infrared region.

3. The device according to claim 2, wherein the centring light beam (34) has a wavelength in the range from 800 to 1,100 nm.

4. The device according to one of the preceding claims, wherein the means for measuring the position (20) of the scattered light/Fresnel reflex of the centring light beam (34) include a camera (36).

5. The device according to claim 4, wherein the camera (36) is connected to a computer (38) which, making use of the measurement data, determines the geometrical centre (42) of the pupil and the position (20) of the scattered light/Fresnel reflex of the centring light beam (34) on the front face of the cornea.

## Revendications

1. Dispositif pour la kératectomie photo-réfractive de l'oeil comprenant
- un faisceau laser UV (UV) pour l'ablation de parties de la cornée,
- un faisceau lumineux de fixation (24), qui a une longueur d'onde dans la plage visible et est orienté sur l'oeil,
- un dispositif pour déterminer le point (20) où le faisceau lumineux de fixation (24) entre par la surface avant de la cornée (12), avec
- un faisceau lumineux de centrage (34), qui est orienté sur le même axe que le faisceau lumineux de fixation (24) sur l'oeil, a une autre longueur d'onde que le faisceau lumineux de fixation et qui génère un réflexe de lumière diffusée/de Fresnel de forme ponctuelle sur la surface avant de la cornée (12), et
- des moyens (28, 36) pour la mesure de la position du réflexe de lumière diffusée/de Fresnel de forme ponctuelle du faisceau lumineux de centrage (34) sur la surface avant de la cornée (12) .

2. Dispositif selon la revendication 1, le faisceau lumineux de centrage (34) ayant une longueur d'onde située dans la zone infrarouge.

3. Dispositif selon la revendication 2, le faisceau lumineux de centrage (34) ayant une longueur d'onde située dans la plage de 800 à 1100 nm.

4. Dispositif selon l'une quelconque des revendications précédentes, des moyens pour la mesure de la position (20) du réflexe de lumière diffusée/de Fresnel du faisceau lumineux de centrage (34) présentant une caméra (36).

5. Dispositif selon la revendication 4, la camera (36) étant raccordée à un ordinateur (38) qui détermine à partir des données de mesure le centre géométrique de la pupille (42) et la position (20) du réflexe de lumière diffusée/de Fresnel du faisceau lumineux de centrage (34) sur la surface avant de la cornée.
